(19) Europäisches Patentamt
European Patent Office
Office européen des brevets

(11) Veröffentlichungsnummer: **0 219 839**
**A2**

(12) # EUROPÄISCHE PATENTANMELDUNG

(21) Anmeldenummer: 86114468.1

(22) Anmeldetag: 18.10.86

(51) Int. Cl.⁴: C 12 P 21/02
C 12 N 15/00, C 12 N 1/20
A 61 K 37/02

(30) Priorität: 22.10.85 DE 3537461

(43) Veröffentlichungstag der Anmeldung:
29.04.87 Patentblatt 87/18

(84) Benannte Vertragsstaaten:
AT BE CH DE ES FR GB GR IT LI LU NL SE

(71) Anmelder: **HOECHST AKTIENGESELLSCHAFT**
**Postfach 80 03 20**
**D-6230 Frankfurt am Main 80(DE)**

(72) Erfinder: **Engels, Joachim, Prof. Dr.**
**Feldbergstrasse 1**
**D-6242 Kronberg/Taunus(DE)**

(72) Erfinder: **Uhlmann, Eugen, Dr.**
**Zum Tablick 31**
**D-6246 Glashütten/Taunus(DE)**

(72) Erfinder: **Wengenmayer, Friedrich, Dr.**
**Am Seyenbach 38**
**D-6238 Hofheim am Taunus(DE)**

(72) Erfinder: **Müllner, Hubert, Dr.**
**Pestalozzistrasse 4**
**D-6233 Kelkheim (Taunus)(DE)**

(72) Erfinder: **Leineweber, Michael, Dr.**
**Heimchenweg 74**
**D-6230 Frankfurt am Main 80(DE)**

(72) Erfinder: **Winnacker, Ernst-Ludwig, Prof. Dr.**
**Dall-Armistrasse 41a**
**D-8000 München 19(DE)**

(72) Erfinder: **Mertz, Roland, Dr.**
**Ursulastrasse 5**
**D-8000 München 40(DE)**

(54) **Derivat des Interleukin-2, seine Herstellung und Verwendung.**

(57) Proteine, deren Aminosäuresequenz im wesentlichen der des natürlichen Interleukin-2 (IL-2) entspricht, wobei jedoch N-terminal die ersten 7 Aminosäuren fehlen, zeigen die biologische Aktivität von IL-2. Diese Proteine können gentechnisch hergestellt werden. Sie eignen sich zur Herstellung von Arzneimitteln mit IL-2-Aktivität.

EP 0 219 839 A2

Derivat des Interleukin-2, seine Herstellung und Verwendung

Human-Interleukin-2, im folgenden IL-2, ist eines der biologisch aktiven Polypeptide, die in letzter Zeit eingehend untersucht wurden, seit sie durch gentechnische Synthesen gut zugänglich wurden. In der europäischen Patentanmeldung mit der Veröffentlichungsnummer (EP-A) 91 539 ist neben der vollständigen Aminosäuresequenz dieses Moleküls auch ein Derivat beschrieben, dem N-terminal die erste Aminosäure fehlt. In der (nicht vorveröffentlichten) deutschen Offenlegungsschrift 3 419 995 ((EP-A 163 249) wurde bereits ein Verfahren vorgeschlagen, mit dem man ein solches Derivat des IL-2 mit Hilfe eines synthetischen Gens herstellen kann.

Überraschenderweise wurde nun gefunden, daß ein Derivat von IL-2 biologisch aktiv ist, bei den N-terminal die ersten 7 Aminosäuren der natürlichen Sequenz fehlen. Die Erfindung betrifft deshalb Derivate von IL-2, bei denen N-terminal diese Aminosäuren nicht vorhanden sind, Verfahren zur Herstellung solcher Derivate, Arzneimittel, die solche Derivate enthalten bzw. die Verwendung dieser Derivate zur Herstellung von Arzneimitteln mit der biologischen Wirkung von IL-2.

Die Erfindung beschränkt sich nicht auf ein IL-2-Derivat mit der natürlichen Aminosäurensequenz, sondern betrifft vielmehr auch Polypeptide, deren Aminosäuresequenz in an sich bekannter Weise verändert wurde, jedoch die biologische Aktivität im wesentlichen erhalten geblieben ist oder sogar verstärkt wurde. Solche Variationen sind beispielsweise aus der EP-A 109 748 bekannt und auch in der deutschen Offenlegungsschrift 3 419 995 vorgeschlagen. Das in dieser Anmeldung vorgeschlagene gentechnische Verfahren beruht auf einer synthetischen DNA-Sequenz, die

eine Vielzahl von singulären Restriktionstellen enthält und somit besonders gut zur Herstellung von Polypeptiden mit veränderter Aminosäuresequenz geeignet ist.

Die erfindungsgemäßen IL-2 Derivate mit der verkürzten Aminosäuresequenz zeichnen sich durch eine Reihe von Vorteilen aus: Im Vergleich zum natürlichen IL-2 zeigt das erfindungsgemäße Derivat mit der verkürzten, aber im übrigen der natürlichen Sequenz entsprechenden Aminosäurefolge eine höhere Expression und damit eine erhöhte Ausbeute. Außerdem ist die Löslichkeit in Wasser auf etwa das Doppelte erhöht und die Retentionszeit in der Hochdruckflüssigkeitschromatographie verkürzt. Diese verbesserten physikalischen Eigenschaften erleichtern die Aufarbeitung, so daß insgesamt die Ausbeute am Reinprodukt weiter erhöht wird.

Unter den gleichen Extraktionsbedingungen zeigt beispielsweise das erfindungsgemäß verkürzte Produkt im Vergleich zum natürlichen IL-2 eine von 10 bis 15 auf bis zu 27 Gew.-% erhöhte Konzentration im Extrakt des ersten Aufarbeitungsschritts. Durch diese verbesserte Löslichkeit wird auch die Renaturierung erleichtert, was ebenfalls zur Erhöhung der Ausbeute an aktivem Reinprodukt beiträgt. Die biologische Aktivität des Produkts entspricht der des Naturprodukts; die genannten Vorteile werden also nicht durch eine Verminderung der Wirksamkeit erkauft.

In den folgenden Beispielen wird die Erfindung näher erläutert. Prozentangaben beziehen sich hierbei auf das Gewicht, wenn keine anderen Angaben gemacht sind.

Beispiel 1

Die DNA-Sequenz I, die für IL-2 kodiert, wird nach Beispiel 5a) der deutschen Offenlegungsschrift 3 419 995 isoliert und mit dem Restriktionsenzym Hinf I nachgeschnitten. Das IL-2-Genfragment, das die Kodons für die Aminosäuren 10 bis 133 umfaßt, wird auf einem 1,5 %igen niedrigschmelzenden Agarosegel abgetrennt und nach Phenolextraktion zurückgewonnen. Dieses Genfragment hat die folgenden DNA-Sequenz (1)

| | | | | | | | | | | |
|---|---|---|---|---|---|---|---|---|---|---|
| | 10 | 11 | | 133 | | | | | | |
| | Thr | Gln | | Thr | Stp | Stp | | | | |
| 5' | ACT | CAA | ... | ACC | TGA | TAG | | | 3' | (1) |
| 3' | | GTT | | TGG | ACT | ATC | AGC | T | 5' | |
| | (Hinf I) | | | | | | (Sal I) | | | |

Die Sequenz (1) wird nun mit dem Linker der DNA-Sequenz (2)

| | | | | | | | | |
|---|---|---|---|---|---|---|---|---|
| | | | Met | Lys | Lys | | | |
| 5' | AA | TTC | ATG | AAA | AAG | | 3' | (2) |
| 3' | | G | TAC | TTT | TTC | TGA | 5' | |
| | (Eco R I) | | | | | (Hinf I) | | |

mittels T 4-DNA-Ligase ligiert. Das Ligationsprodukt wird mit EcoR I und Sal I nachgeschnitten und gemäß Beispiel 5a) der deutschen Offenlegungsschrift 3 419 995 in das mit den gleichen Enzymen geöffnete Expressionsplasmid pKK 177.3 eingebaut. Man erhält das Hybridplasmid mit der Bezeichnung p IL-2/2, das dem Plasmid p 159/6 (Figur 5 der deutschen Offenlegungsschrift 3 419 995) entspricht, wobei lediglich die Sequenz "IL II" durch die erfindungsgemäße verkürzte DNA-Sequenz ersetzt ist, die durch Ligieren der vorstehend genannten DNA-Sequenzen (1) und (2) erhalten wird.

Wird diese erfindungsgemäß verkürzte Sequenz gemäß Beispiel 5b) (Figur 6) der genannten deutschen Offenlegungsschrift in das Expressionsplasmid p Trp H I eingebaut, so erhält man das Plasmid p IL-2/3.

Beispiel 2

Die Hybridplasmide p IL-2/2 bzw. p IL-2/3 werden in bekannter Weise in den handelsüblichen Stamm E. coli W 3110 transformiert und in YT-Medium (J. Miller, Experiments in Molecular Synthesis, Cold Spring Harbor, 1972) über Nacht angezogen. Die Kultur wird dann im Gewichtsverhältnis 1 zu 20 in Minimal-A-Medium (J. Miller, a. a. O.) überimpft und bis zu einer optischen Dichte von 0,5 bei 600 nm im Schüttelkolben (260 Upm) und 37°C angezogen. Anschließend wird die Expression bei den mit p IL-2/2 transformierten Bakterien mittels Isopropyl-ß-D-thiogalacto-pyranosid und bei den mit p IL-2/3 transformierten Bakterien mit Indolylacrylessigsäure induziert, die Kulturen mit 1 % Glucose "nachgefüttert" und weitere 3 Stunden bei 37°C geschüttelt.

Gleiche Anteile der Kulturen werden dann mit Puffer (7 M Harnstoff, 1 % Natriumdodecylsulfat (SDS), 4 % Mercaptoethanol, 30 mM Tris, pH 6,8) versetzt, 5 Minuten gekocht und die Proteine auf 15 %igen SDS-Acrylamidgelen aufgetrennt. Durch Anfärben mit ®Coomassie-Brillant-Blau und anschließend Entfärbung des Gels werden die aufgetrennten Proteine sichtbar gemacht.

Ein Vergleich der Proteine, die unter den gleichen Bedingungen mit dem Plasmid p 159/6 (Figur 5 der deutschen Offenlegungsschrift 3 419 995) erhalten werden, zeigt, daß erfindungsgemäß mehr Protein mit IL-2-Aktivität gebildet wird.

Patentansprüche

1. Protein mit der biologischen Aktivität des Human-Interleukin-2 (IL-2), dadurch gekennzeichnet, daß seine Aminosäuresequenz im wesentlichen der des natürlichen IL-2 entspricht, jedoch die ersten 7 Aminosäuren der natürlichen Sequenz am N-terminalen Ende fehlen.

2. Protein der Formel

Met-(IL-2 8-133)

in der (IL-2 8-133) die Aminosäuren 8-133 von IL-2 bedeutet.

3. DNA, die für ein Protein nach Anspruch 1 oder 2 kodiert.

4. Hybridvektor, der eine DNA nach Anspruch 3 enthält.

5. Wirtszelle, die einen Vektor nach Anspruch 4 enthält.

6. E. coli, enthaltend einen Vektor nach Anspruch 4.

7. Verfahren zur Herstellung eines Proteins nach Anspruch 1 oder 2, dadurch gekennzeichnet, daß man in einer Wirtszelle nach Anspruch 5 oder 6 das gewünschte Protein exprimiert.

8. Arzneimittel, gekennzeichnet durch einen Gehalt an einem Protein nach Anspruch 1 oder 2.

9. Verwendung der Proteine nach Anspruch 1 oder 2 zur Herstellung von Arzneimitteln mit IL-2-Aktivität.

Patentansprüche Österreich, Spanien und Griechenland:

1. Verfahren zur Herstellung eines Proteins mit der biologischen Aktivität des Human-Interleukin-2 (IL-2), dadurch gekennzeichnet, daß man in einer Wirtszelle ein Protein exprimiert, dessen Aminosäuresequenz im wesentlichen der des natürlichen IL-2 entspricht, wobei jedoch die ersten 7 Aminosäuren der natürlichen Sequenz am N-terminalen Ende fehlen.

2. Verfahren nach Anspruch 1, dadurch gekennzeichnet, daß die Wirtszelle E. coli ist.

3. Verfahren nach Anspruch 1 oder 2, dadurch gekennzeichnet, daß das Protein der Formel

Met-(IL-2 8-133)

exprimiert wird, wobei (IL-2 8-133) die Aminosäuren 8-133 von IL-2 bedeutet.